Europäisches Patentamt

European Patent Office

Office européen des brevets

(11) Publication number: **0 235 815**
**A2**

# EUROPEAN PATENT APPLICATION

(21) Application number: 87103116.7

(51) Int. Cl.4: **A41B 13/02**

(22) Date of filing: 05.03.87

(30) Priority: 05.03.86 US 836352

(43) Date of publication of application:
09.09.87 Bulletin 87/37

(84) Designated Contracting States:
DE FR GB IT NL SE

(71) Applicant: KIMBERLY-CLARK CORPORATION
401 North Lake Street
Neenah Wisconsin 54956(US)

(72) Inventor: Donnelly, Harold Frederick
1096 Lake Shore Drive
Menasha Wisconsin(US)
Inventor: Plaia, Lawrence Paul
1920 East Pershing Street
Appleton Wisconsin(US)
Inventor: Steger, Paul Stephen
224 Stevens Street
Neenah Wisconsin(US)

(74) Representative: Diehl, Hermann Dr. et al
Diehl & Glaeser, Hiltl & Partner
Flüggenstrasse 13
D-8000 München 19(DE)

(54) A method for attaching multi-strand elastic material to a garment, an apparatus for carrying out this process and the garment made therewith.

(57) The present invention provides an improved method and apparatus for attaching multi-strand elastic material to a selected portion of a garment - (10). A supplying mechanism provides an elastic material which has been oriented to a selected elongation and has been separated into a plurality of individual elastic strands (20). The separated elastic strands (20) are adhered to a backsheet (12) and liner sheet (14) of the garment (10), and occupy an elastic strand region having a selected transverse, cross-directional width dimension. A band (18) of adhesive is applied to at least one of the backsheet - (12) and liner sheet (14) for bonding the elastic strands (20) to the garment (10). This adhesive band (18) has a cross-directional width which is not less than the cross-directional width of the elastic strand region. The garment (10) produced in accordance with the invention exhibits high garment integrity and good elastic performance.

FIG. 2

# A METHOD FOR ATTACHING MULTI-STRAND ELASTIC MATERIAL TO A GARMENT, AN APPARATUS FOR CARRYING OUT THIS PROCESS AND THE GARMENT MADE THEREWITH

The present invention relates to a multi-strand elastic for shirring selected portions of an article, such as a disposable garment. More particularly, the present invention relates to an improved process for applying multi-strand elastic to an article in a configuration which maintains increased integrity of the article and provides improved elastic performance.

Various techniques have been employed to attach multiple strands of elastic material to a flexible substrate to shir the substrate and form elasticized puckers. For example, U. S. Patent 3,575,782 issued April 20, 1971 to P Hansen describes an elastic web comprising multiple elastic strands attached to a nonwoven, porous fibrous web. The elastic filaments are attached in an elongated condition to the nonwoven web with a binder mixture that impregnates the web. The assembled structure is then heated to relax the structure sufficiently to permit the elastomeric yarns to retract and produce the desired degree of shirring.

Other techniques have been employed to form elasticized bands on a disposable garment. For example, see U. S. Patent 4,486,192 issued December 4, 1984 to W. Sigl; U. S. Patent 4,352,355 issued October 5, 1982 to F. Mesek, et al.; and U. S. Patent 4,388,075 issued June 14, 1983 to F. Mesek, et al.

In addition, various configurations have been employed for the ribbons employed to shir the garments. In particular, U. S. Patent 4,300,562 issued November 17, 1981 to H. Pieniak describes an elastic which has a grid-like structure. U. K. Patent Application GB 2 118 021 A published October 26, 1983 by Suzuki, et al. describes elastic filaments which are partially interjoined.

European Patent Application 0 121 178 A1 published October 10, 1984 of R. DeJonckheere describes a device for attaching multiple elastic strands to a film substrate. The strands are slit from a relatively wide ribbon and are stretched by varying amounts prior to being attached to the film.

Conventional techniques for securing multiple strands of elastic material to selected portions of a garment have not been completely satisfactory because the multiple elastic strands do not reliably remain secured to the garment. The individual strands often separate from the garment at random locations and cause an unsightly appearance. In addition, this separation of the elastic strands reduce the shirring effect on the garment and decreases the functionality of the elasticized bands. The separation of the elastic strands from the garment are particularly pronounced when the garment is stored at elevated temperatures above about 55°C. At these elevated temperatures, the bonds between the elastic strands and the garment substrate can soften and allow the elastic strands to contract without producing any further shirring of the garment. This reduces the tension in the shirred portions of the garment when the gathers are stretched in use. This undesirable phenomenon is commonly referred to as "creep".

It is an object of this invention to provide a simple but effective method and apparatus for attaching multi-strand elastic material to parts of a garment. The method of this invention to solve this object is described in claim 1. Further advantageous features of this process are evident from the dependent claims. The corresponding apparatus of this invention is described in independent claim 7. Further advantageous features features of this apparatus are evident from the dependent claims 8-11. The invention further provides an improved garment as described in claim 12. Further advantageous features of this garment are evident from the dependent claims 13 to 20.

The present invention according to one aspect refers to an improved method and apparatus for attaching multi-strand elastic material to a selected portion of a garment. Generally stated, the apparatus includes supplying means for providing an elastic material which has been oriented to a selected elongation and has been separated into a plurality of elastic strands. Attaching means adhere the separated elastic strands to a liner sheet and backsheet of the garment. The attaching means includes an applicator means which forms a band of adhesive on at least one of the backsheet and liner sheet of the garment. A pressuring means secures the elastic strands to the garment liner and backsheet.

In particular aspects of the invention, the apparatus configures the separated elastic strands to occupy an elastic strand region having a selected cross-directional width dimension. In addition, the applicator means forms on the liquid permeable liner a band of adhesive having a cross-directional width which is not less than the width of the elastic strand region.

The invention further provides an improved method for attaching multi-strand elastic material to a selected portion of a garment. The method includes the steps of supplying an elastic material which has been oriented to a selected elongation and has been separated into a plurality of individual elastic strands. The separated elastic strands are adhered to a liner sheet and backsheet of the garment employing a configuration wherein a band of adhesive is formed on at least one of the liner sheet and backsheet of the garment. These elastic strands are secured to the liner sheet and backsheet with an applied pressure.

The distinctive method and apparatus of the invention are employed to provide an improved garment comprising a backsheet, and a liner sheet in adjacent, facing relation with the backsheet. A set of multiple strands of elastic material is attached to selected portions of the backsheet and liner sheet, and occupies an elastic strand region having a selected transverse cross-directional width dimension. A band of adhesive, which is applied to one or both of the liner sheet and backsheet, bonds the elastic strands to the garment and has a cross-directional width which is not less than the cross-directional width of the elastic strand region. These attached elastic strands operate to shir portions of the article. In particular aspects of the invention, the attached elastic strands exhibit a creep value of not more than about 40mm at each end of the strands.

The distinctive garment produced in accordance with the method and apparatus of the invention advantageously has increased mechanical integrity and improved performance. The multiple elastic strands, when fitted about a selected portion of a wearer, distribute the applied pressure and stress over a larger contact area, and thereby reduce undesired marking on the skin of the wearer. In addition, the elastic strands remain reliably attached to the garment substrate to maintain the desired shirring effect and to provide a more pleasing appearance. The integrity of the garment is maintained even at elevated temperatures and the garment can be more readily stored without degrading its performance characteristics. In particular, the improved garment of the invention has multiple elasticized strands bonded to the garment with a technique and configuration that provides increased resistance to creep.

The invention will be more fully understood and further advantages will become apparent when reference is made to the following detailed description of the invention and the drawings, in which:

Fig. 1 shows a schematic representation of the apparatus for affixing elastic strands to a portion of a garment;

Fig. 2 representatively shows a disposable diaper which incorporates the multi-strand elastic of the invention;

Fig. 3 representatively shows a disposable diaper in which the multiple elastic strands have gathered the marginal edge portions of the garment;

Fig. 4 representatively shows a cross-sectional view taken along section 4-4 of Fig. 2.

Fig. 5 representatively shows a diaper garment being marked for testing to determine the creep value of the elastic strands; and

Fig. 6 shows a representative test sample mounted for testing.

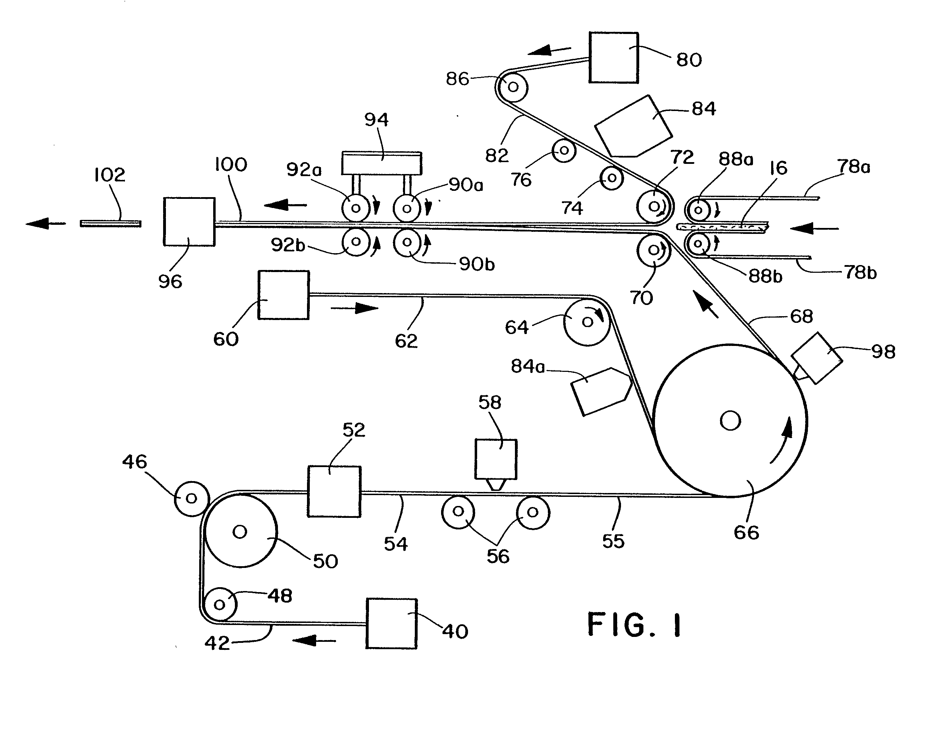

Referring to Fig. 1, the apparatus of the invention includes elastic supplying means, such as elastic supply 40 for providing an elastic material 54 which has been oriented to a selected elongation by stretching means, such as differentially driven rollers 46 and 48. In addition, the elastic material has been separated into a plurality of elastic strands by processing means, such as slitting device 52. Attaching means, such as adhesive applicator 58, adhesive applicator 84 and/or adhesive applicator 84a, and attachment roller 66, adhere the separated elastic strands 55 directly to a backsheet material 62 and a liner sheet material 82 of the garment. The attaching means includes applicator means 84 and optional applicator 84a for forming a band of adhesive on at least one of the backsheet 62 and liner sheet 82 materials. In the shown embodiment, adhesive applicator 58 applies an adhesive to adhere elastic strands 55 to the backsheet material 62, and adhesive applicator 84, forms an extended, substantially continuous band of adhesive only on a liner sheet 82 of the garment. In an alternative embodiment of the invention, the optional adhesive applicator 84a may form an extended, substantially continuous band of adhesive onto backsheet material 62. Pressuring means, such as nip roller pairs 90a-b and 92a-b, secure the elastic strands to garment liner 82 with an applied pressure directed against the adhesive bonds.

In the shown embodiment of the invention, elastic supply 40 provides a strip or ribbon 42 of a suitable elastomeric material. Such elastic materials include, for example, natural rubber and synthetic elastomers, such as polyurethane elastomer.

Orienting means stretch strip 42 to impart a selected elongation to the elastic material. Typically, the amount of elongation ranges from about 20% to 150%. In the shown embodiment of the invention, the orienting means comprises traction roller 48 and stretching rollers 46 and 50.

Traction roll 48 is rotatably driven by suitable driving means, such as an electric motor (not shown), to frictionally engage and draw elastic strip 42 from supply 40 to a selected withdrawal speed. Ribbon 42 passes through the nip between counterrotating rollers 46 and 50, which are also rotatably driven by suitable driving means. Stretching rollers 46 and 50 frictionally engage ribbon 42 in the nip therebetween to move the engaged portion of the ribbon at a second, stretching speed that is greater than the withdrawing speed provided by roller 48. As a result, the length of ribbon 42 between stretching roller 46 and traction roller 48 becomes stretched and elongated by a selected amount. The amount of elongation is determined in part by the magnitude of the difference between the stretching speed and the withdrawing speed. In the shown embodiment of the invention, the stretching speed is about 10-100% greater than the withdrawing speed.

Processing means 52 separates the ribbon of elastic material to form a set comprising a plurality of elastic strands 54. For example, conventional slitting means can be employed to cut strip 42 into a plurality of individual, elastic strands to provide strand set 54. In the shown embodiment, strip 42 is slit into a strand set 54 comprising four individual elastic strands having generally rectangular cross-sections. It is readily apparent, however, that the apparatus could be configured to slit strip 42 into a different number of multiple strands.

A suitable backsheet supplying means 60 provides a substantially continuous web of backsheet material 62. The backsheet material is typically a liquid impervious material, such as a polyolefin film or the like. Suitable polyolefin films includes, for example, polyethylene and polypropylene. Guide roller 64 directs backsheet material 62 toward assembling roller 66. As elastic strands 54 move from slitter 52, supporting rollers 56 guide the strands past an adhesive applicator 58 in a configuration wherein the individual strands are separated from each other by a selected distance. For example, rollers 56 can include parallel circumferential grooves formed in the outer peripheral surfaces thereof. The separation distances between the grooves are arranged and configured to establish the desired spacing distance between the individual elastic strands. In addition, the individual elastic strands can be passed through a comb device which maintains the separation between the strands.

Adhesive applicator 58 is controlled to selectively apply an adhesive, such as a hot melt, pressure-sensitive adhesive to those portions of elastic strands 54 that are appointed for attachment to backsheet material 62. Suitable hot melt, pressure-sensitive adhesives include, for example, Ecomelt H-345 and H-314 sold by Ecomelt AG of Sursee, Switzerland; FULLER HB-1351 sold by H. B. Fuller Adhesives of Vadnais Heights, Minnesota; and Findley 9917-372 sold by Findley Adhesives of Elm Grove, Wisconsin.

The set of adhesive coated elastic strands 55 move onto assembling roll 66 and wrap arounds its over peripheral surface. As representatively shown in Fig. 1, the adhesive coated elastic strand set 55 becomes positioned onto backsheet material 62 and becomes adhesively attached to the backsheet material along the selected portions of the elastic strands that have been previously coated with adhesive. The assembly 68 composed of backsheet material 62 and elastic strands 55 move from assembling roll 66 and are directed around guide rolls 70 for further assembly to a liner sheet material 82.

In an optional, alternative embodiment of the invention an auxiliary adhesive applicator 84a may be employed to deposit an extended, generally uniform and substantially continuous band of adhesive, such as hot melt pressure-sensitive adhesive, onto appointed portions of backsheet material 62. The applied adhesive band is adjusted to have a transverse, cross-directional width which is greater than or equal to the cross-directional width of the space occupied by the elastic strand region. However, care must be taken to avoid the transfer of excessive heat from the adhesive to the backsheet material. Excessive heat can undesirably distort or melt the backsheet material. When adhesive applicator 84a is employed, adhesive applicator 58 may be deleted.

Liner supply 80 provides a substantially continuous web of liner material 82, which is directed by suitable guiding means, such as roller 86, toward liner support rollers 76 and 74. The support rollers position liner material 82 as it passes by adhesive applicator 84.

Adhesive applicator 84 deposits an extended, generally uniform, and substantially continuous band of adhesive, such as hot melt, pressure-sensitive adhesive, onto selected portions of liner sheet material 82. Suitable adhesives include, for example, the hot melt adhesives previously described.

The applied adhesive band is selectively adjusted to have a transverse, cross-directional width which is greater than or equal to the cross-directional width of the space occupied by the separated elastic strands on assembled web 68. After the application of the adhesive band to liner sheet material 82, the liner sheet material is directed around guide roller 72 for further assembly to web 68.

Liner sheet material 82 and web 68 are directed to pressuring means for establishing the desired attachment bond formed by the band of adhesive between the elastic strands and the garment liner sheet material. In the illustrated embodiment, the pressuring means comprises nip roller pairs 90a-b and 92a-b. Liner sheet material 82 and web 68 pass into the nip between the roller pairs and are suitably pressed together to operably increase the bonding strength and integrity between the elastic strands and the liner sheet material 82. A regulator 94 controls the pressure applied by the nip roller pairs to assure an intimate contact between the elastic strands and the adhesive band on liner sheet material 82. In a particular aspect of the invention, the pressure applied by each pair of nip rollers is about 50 psi (345 kPa), preferably ranges from about 50 -70 psi (345 -483 kPa), and more preferably is about 60 psi (414 kPa) to provide improved performance.

When producing a disposable absorbent garment, such as a disposable diaper, incontinent garment, sanitary napkin or the like, an absorbent body 16 is interposed and assembled between liner sheet material 82 and web 68. In the shown embodiment, an assembly means for inserting an absorbent body between the backsheet material and the liner sheet material comprises a pair of codirectionally moving hugger belt mechanisms 78a and 78b, which are guided by suitable support rollers 88a and 88b, respectively. The hugger belts carry a selected series of absorbent bodies therebetween and insert them at selected intervals and locations between the backsheet material and the liner sheet material. Adhesive applicator 98 applies a selected pattern of adhesive to web 68 to bond and secure the absorbent body 16 thereto. In addition, additional converting means 96 contour and cut the composite assembly 10 into a plurality of discrete individual garment articles. In this converting process, the elastic strands are allowed to contract from their elongated condition to operably shir the portions of the garment to which those strands are attached.

The above description of the method and apparatus of the invention has been made in the context of applying and affixing a single, closely spaced set of elastic strands to selected portions of a garment. It will be readily apparent that a plurality of distinct sets of multiple elastic strands may be affixed to the garment by employing parallel arrangements of selected components of the apparatus. These parallel arrangements could be operated simultaneously or sequentially. For example, if one desired to elasticize two lateral side margins of a disposable diaper, the orienting means of the invention could be employed to simultaneously stretch two spaced apart, parallel strips or ribbons of elastic material 42. Slitting means would cut each strip into a set 54 of individual strands and each strand set would be processed with adhesive from applicator 58 to provide two coated elastic strand sets 55. Both strand sets could then be applied to backsheet material 62 on assembly roll 66. In similar parallel fashion, adhesive applicator 84 would apply two adhesive bands on liner sheet material 82 in a corresponding registration with the elastic strand sets 55. Additional sets of pressurizing nip rollers similar to 90a-b could be employed to form attachment bonds between the two elastic strand sets 55 and their corresponding adhesive bands that have been applied to liner sheet material 82.

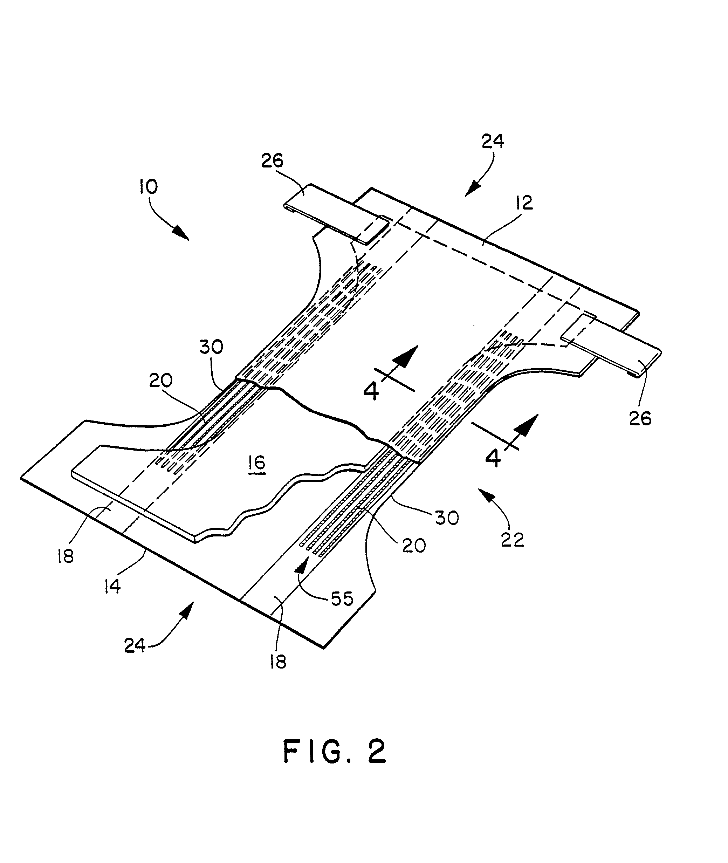

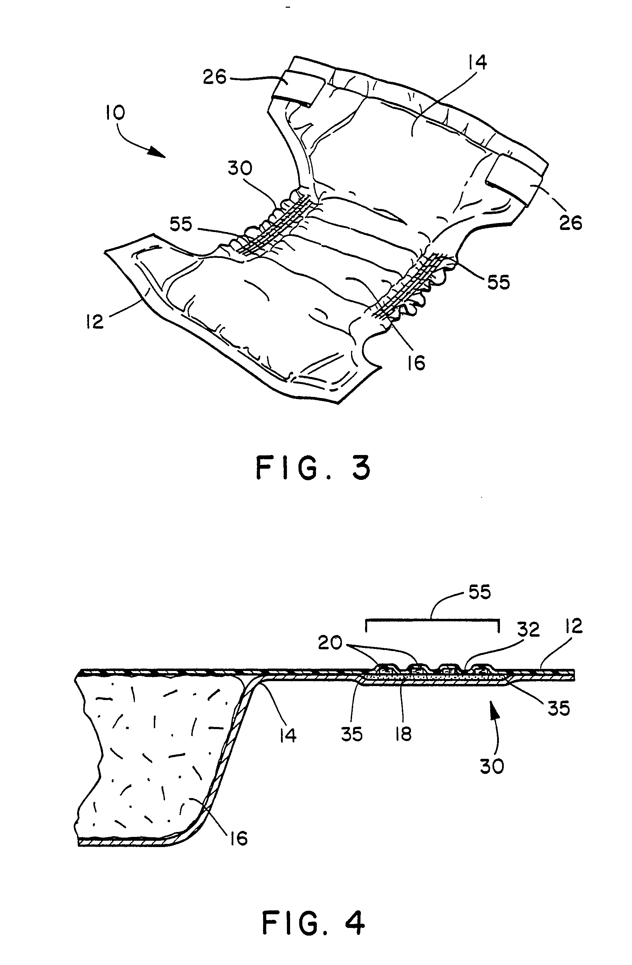

Figs. 2 and 3 illustrate a representative disposable diaper 10 which has been constructed in accordance with the present invention. While the context of the following detailed description is made with respect to a disposable diaper, it will be readily apparent to a person having ordinary skill in the art that the present invention can also be employed to apply multi-strand elastics to shir other articles and garments. For example, such multi-strand elastics can be applied to elasticize incontinence garments, sanitary napkins, wrist cuffs, hat bands, waistbands and the like.

Disposable diaper 10 is generally "hour-glass" or I-shaped, and comprises two waistband sections 24 at its longitudinal end portions. A narrower intermediate portion 22 interconnects the waistbands 24 and includes a marginal side edge portion 30 at each of the two lateral sides of the diaper. The intermediate portion also includes a crotch section that is constructed and arranged to fit between the legs of the wearer. The diaper garment further comprises an inner liner sheet 14, an outer backsheet 12 located in an adjacent, facing relation with said liner sheet, and an absorbent body 16 located between and in facing relation with the liner sheet and backsheet. Each of the liner sheet, backsheet and absorbent body delimits two waistband portions located at its longitudinal ends and delimits an intermediate portion that interconnects the waistband portions. The intermediate portion of each of the liner sheet, backsheet and absorbent body further defines a respective crotch section which is constructed and arranged to fit between the legs of the wearer.

Liner sheet member 14 forms the body facing surface of the garment and is composed of a liquid permeable material, such as spunbonded polypropylene and/or polyethylene, thermally bonded carded webs, resin bonded carded webs and/or dry formed webs of natural and synthetic fibers. Since this liner sheet contacts the infant's skin, it should be composed of a soft and nonabrasive material.

Absorbent body 16 is assembled and optionally secured to liner 14 with suitable attachment means, such as a selected pattern of adhesive. For example, a plurality of adhesive lines can be employed to secure absorbent body 16 to liner 14. Typically, absorbent body 16 is composed of a cellulosic material commonly referred to "fluff". The absorbent body is sized to be smaller than liner 14, particularly in the lateral, cross-direction to allow the formation of flexible and gatherable gasket regions at the marginal edges 30.

In the shown embodiment, two substantially continuous and generally rectilinear adhesive bands 18 are applied to liner 14 of diaper 10. One adhesive band is located adjacent to each of the lateral side edges of absorbent body 16 and intermediate the associated, terminal side edges of the liner and the absorbent body. If desired, the lengthwise extent of adhesive bands 18 can be restricted to the intermediate region or the crotch section of liner 14. Alternatively, the adhesive bands can extend completely to the longitudinal ends of the liner, as shown in the embodiment of Fig. 1. Adhesive bands 18 advantageously strengthen the liner material in the region of the individual elastic strands 20, and help enable the liner material to withstand stresses imposed by the elastic. Adhesive band 18 also helps to maintain and hold the desired positioning and configuration of the elastic strands within the garment, particularly in the cross-direction. The transverse, cross-directional width of adhesive band 18 is at least as wide as the cross-directional width of the space occupied by the set 55 of multiple, aligned strands 20 at a particular diaper edge. Preferably, the cross-directional width of adhesive band 18 is greater than the width of the elasticized region occupied by a set of multiple strands to provide improved performance.

In a particular aspect of the invention, the cross-direction width of elastic strand set 55 is at least about 10mm, preferably ranges from about 10mm -25mm, and more preferably is about 19mm to provide improved garment performance. In particular, when the cross-directional width of the space occupied by elastic strand set 55 is at least about 10 mm, the diaper garment exhibits and increased ability to contain absorbed liquids and prevent leakage.

Backsheet 12 is generally coextensive with liner sheet 14 and is typically bonded to both absorbent body 16 and liner sheet 14, as well as to each of the two sets of elastic strands 55. For example, the backsheet can be adhesively attached to the absorbent body with lines of hot melt pressure-sensitive adhesive, and can be attached to the overlapping marginal edge sections of the liner sheet with other lines of hot melt, pressure-sensitive adhesive.

Fastening means, such as conventional pressure-sensitive adhesive tape tabs 26, are employed to secure the diaper around the body of an infant. It will be readily apparent that various othe types of fastening means, such as straps, buttons, snaps and the like may be employed to secure the garment.

Referring to Fig. 4, adhesive band 18 may also operate to secure backsheet 12 to liner 14. For example, the extreme lateral side edges 35 of adhesive band 18 may effectively bond portions of backsheet 12 and liner sheet 14 together. In addition, backsheet 12 and liner sheet 14 may be bonded together at the portions 32 thereof that are located between two adjacent elastic strands.

When elastic strands 20 are attached to liner 14 and backsheet 12 in accordance with the invention, the elastic strands operably shir and gather the marginal edges of the diaper crotch section to form a stretchable, elasticized gasket which conformably fits around the legs of an infant when the diaper is worn. The spaced-apart strands spread out the force applied against the infant's skin and reduce the likelihood of skin marking. In addition, the diapers can be more readily stored with less likelihood of having the elastic strands lose their gasket-forming tension within the garment. During storage, the elastic strands maintain their attachments within the diaper and exhibit less creep.

The method and apparatus of the present invention are advantageously capable of producing a garment having multiple elastic strands attached in a configuration which provides a creep value of less than about 40mm measured after an exposure to a temperature of about 100°F (about 38°C). Preferably, the creep value is less than about 25mm and more preferably is less than about 10mm to provide improved product performance.

The following procedure is employed to measure the creep value of a set of elastic strands attached to a garment:

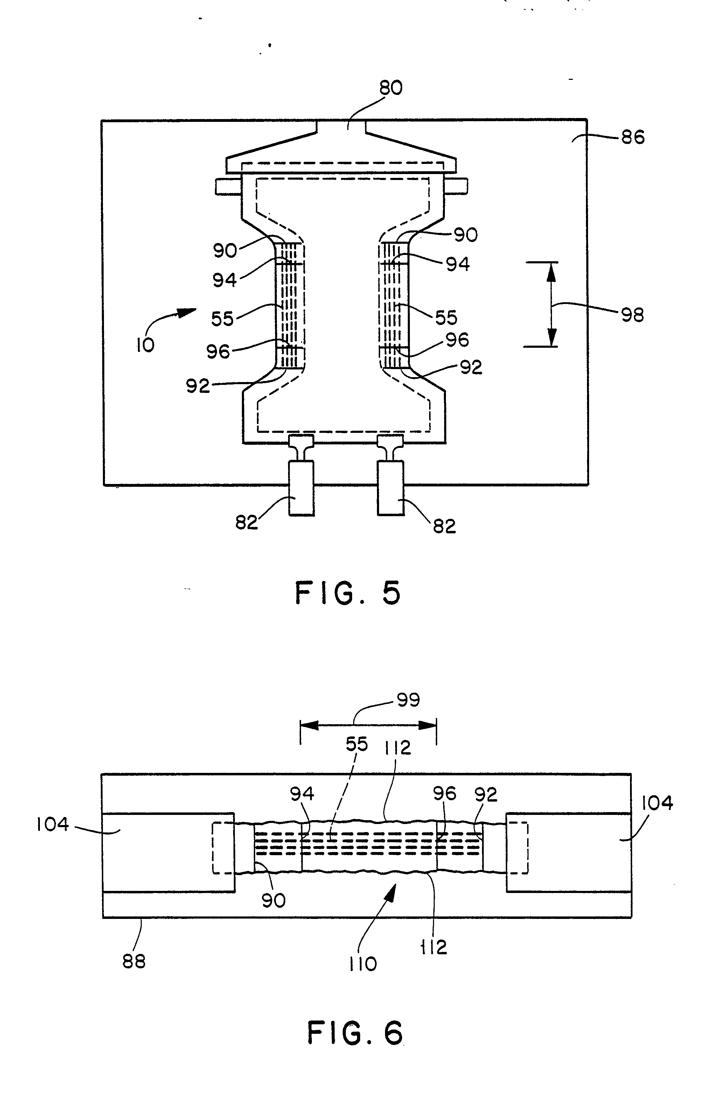

The garment is first aged at least 4 hours after it has been manufactured. Then, as illustrated in Fig. 5, the garment such as the shown diaper 10, is suspended vertically from a support 86 by clip 80. The clip is arranged so as not to contact any portions of elastic strand sets 55. The terminal ends of the adhesive attachments of the elastic strands to the diaper backsheet and liner sheet are identified with ink marks 90 and 92. Two weights 82 weighing 500gm each are then suspended from the diaper with suitable clips which are attached in vertical alignment with the elastic strand sets 55. Two reference measurement points are identified on each of the strand sets with marks 94 and 96 which are spaced apart by a distance 98 measuring 7in (17.8 cm). Again, the clips employed to attach the weights are arranged so as to not contact any portions of the elastic strand sets 55.

The garment is then removed from support 86, and test samples 100 are cut from the garment. The samples include all of the component layers of the garment, and are configured with the lateral side edges of elastic strand set 55 spaced 0.25-0.5 in (0.635-1.27cm) from the lateral, cut edges 102 of the sample. The sample is then mounted on a rigid sample support 88 composed of a suitable material, such as metal or plastic. The sample is mounted in a stretched condition with the reference marks 94 and 96 stretched apart by a distance 99 that measures 5 63/64 in (15.2cm). The sample is held in this stretched condition with high adhesion-strength adhesive tape 104 or other securing means capable of holding the ends of the test sample immobile on support 88 during the remainder of the test. The tape should not contact the elastic strands. The mounted sample is then placed in an oven heated to a temperature of 100°F, +2°F, -3°F (37.8°C, +1.1°C, -1.7°C). The sample is held in this oven for a time period of 90 min if the elastic strands are composed of a synthetic elastomer, and for a time period of 150 min if the elastic strands are composed of a natural rubber. The sample is then removed from the oven, and the distances that the terminal ends of plastic strand set 55 have retracted away from each of the initial identifying marks 90 and 92 are measured and recorded. The amount of retraction or creep measured from each of the marks 90 and 92 should not be more than 40mm.

Having thus described the invention in rather full detail, it will be readily apparent to a person having ordinary skill in the art that various changes and modifications can be made without departing from the spirit of the invention. All of such changes and modifications are contemplated as being within the scope of the invention as defined by the following claims.

## Claims

1. A method for attaching multi-strand elastic material to a selected portion of a garment, comprising the steps of:

    a. supplying an elastic material, which has been oriented to a selected elongation and has been separated into a plurality of individual elastic strands;

    b. adhering said separated elastic strands to a backsheet and liner sheet of said garment, wherein said adhering step includes forming a band of adhesive on at least one of said backsheet and liner sheet of said garment; and

    c. securing said elastic strands to said backsheet and liner sheet with an applied pressure.

2. A method as recited in claim 1, wherein said securing step (c) includes the step of forcing said elastic strands against said band of adhesive with a pressure of at least about 50 psi (345 kPa).

3. A method as recited in claim 1 or 2, wherein said adhering step (b) includes forming a band of adhesive only on said liner sheet of said garment.

4. A method as recited in one of the preceding claims, wherein said adhering step (b) comprises the step of applying an adhesive of said elastic strands to bond said elastic strands to said backsheet.

5. A method as recited in one of the preceding claims, wherein said adhered and secured elastic strands are thereby operable to shir said backsheet and liner sheet, and operable to provide a creep value of not more than about 40 mm.

6. A method as recited in one of the preceding claims, further comprising the steps of:

    a. selectively controlling the amount of cross-directional separation between said separated elastic strands to deine a strand region having a cross-directional width occupied by said elastic strands; and

    b. adjusting said band of adhesive to have a cross-directional width which is not less than the cross-directional width of said strand region.

7. An apparatus for attaching multi-strand elastic material (54) to a selected portion of a garment, comprising:

    a. supplying means (40) for providing an elastic material (42) which has been oriented - (46,48,50) to a selected elongation and has been separated (52) into a plurality of elastic strands (54);

    b. attaching means (58;84,84a) for adhering said separated elastic strands to a backsheet (62) and liner sheet (82) of said garment, said attaching means including applicator means (84,84a) for forming a band of adhesive on at least one of said backsheet (62) and liner sheet (82) of said garment; and

    c. pressuring means (90a,90b;92a,92b;94) for securing said elastic strands (54) to said garment liner (82) with said band of adhesive.

8. An apparatus as recited in claim 7, wherein said applicator means (84) forms said band of adhesive only on said liner sheet (82).

9. An apparatus as recited in claim 8, wherein said attaching means (58) applies an adhesive onto said separated elastic strands (54) to adhere said strands (54) to said backsheet (62).

10. An apparatus as recited in one of claims 7-9, wherein said pressuring means (94) provides an attaching pressure of at least about 50 psi (345 kPa).

11. An apparatus as recited in one of claims 7-10, further comprising assembly means - (78a,78b;88a,88b) for inserting an absorbent body - (16) between said backsheet (62) and said liner sheet (82).

12. A garment (10) comprising:
a backsheet (12);
a liner sheet (14) in adjacent, facing relation with said backsheet (12);

a set of multiple strands (20) of elastic material attached to selected portions of said backsheet - (12) and liner sheet (14), and occupying an elastic strand region having a selected transverse cross-directional width dimension; and

a band (18) of adhesive located on at least one of said backsheet (12) and liner sheet (14) for bonding said elastic strands (20) to said sheets (12,14), said adhesive band (18) having a cross-directional width which is not less than the cross-directional width of said elastic strand (20) region.

13. A garment as recited in claim 12, further comprising an absorbent body (16) located between and in facing relation with said backsheet - (12) and said liner sheet (14).

14. A garment as recited in claim 12 or 13, wherein said liner sheet (14) is composed of a liquid permeable material.

15. A garment as recited in one of claims 12-14, wherein said band (18) of adhesive is located only on said liner sheet (14).

16. A garment as recited in one of claims 12-15, wherein said attached elastic strands (20) shir portions of said article and have a creep value of not more than about 40 mm at each end of the strands after an exposure to a temperature of about 100°F (about 38°C).

17. A garment as recited in one of claims 12-16, wherein:

said backsheet (12) has two waistband end portions (24) and has an intermediate portion (30) which interconnects said waistband portions (24) and defines a backsheet crotch section;

said liner sheet (14) has two waistband end portions (24) and has an intermediate portion (30) which interconnects said liner waistband portions - (24) and defines a liner crotch section;

one of said set of multiple elastic strands (20) is located at each of two marginal side edge regions - (30) of the garment along at least the crotch sections of said backsheet (12) and said liner sheet - (14); and

one of said bands (18) of adhesive bonds each of said set of elastic strands (20) to said liner sheet - (14).

18. A garment as recited in claim 17, wherein each of said attached sets of elastic strands (20) has a creep value of not more than about 40mm after an exposure to a temperature of about 100°F (about 38°C) for a time period of 150 min.

19. A garment as recited in one of claims 12-18, wherein said backsheet (12) is composed of a substantially liquid impervious material and said liner (14) is composed of a liquid pervious material.

20. A garment as recited in claim 19, wherein said band of adhesive (18) is located only on said liner sheet (14).

K 4650-EP

FIG. I

FIG. 2

FIG. 3

FIG. 4

FIG. 5

FIG. 6